# FASCICULE DE BREVET EUROPEEN

(11) **EP 0 359 844 B1**
(45) Date de publication et mention de la délivrance du brevet: **21.07.1993**
(21) Numéro de dépôt: 88115354.8
(22) Date de dépôt: 19.09.1988
(51) Int. Cl.: C12N 5/00, A61K 35/78

(54) **Procédé de production de substances d'origine végétale**
Verfahren zur Produktion von PflanzensubstanzEN
Process for the production of plant materials

(43) Date de publication de la demande: 28.03.1990
(73) Titulaire: SOCIETE DES PRODUITS NESTLE S.A., 1800 Vevey (CH)
(72) Inventeur: De March, Ghislaine, F-37540 St-Cyr Sur Loire (FR); Hariel, Jean, F-74100 Vetraz-Monthoux (FR); Petiard, Vincent, F-37100 Tours (FR)

(56) Documents cités:
- DE-A- 824 935
- DE-A- 3 138 493
- BIOLOGICAL ABSTRACTS, vol. 83, no. 4, 1987, page 912, résumé no. 37813, Philadelphia, PA, US; J.M. AUGEREAU et al.: "Long term storage of callus cultures at low temperatures or under mineral oil layer", & PLANT CELL REP 5(5), 372-376, 1986
- BIOLOGICAL ABSTRACTS/RRM, vol. 34, 1988, résumé no. 34106652; V. PETIARD: "In-vitro plant cell cultures present development and prospects", & ANNALES PHARMACEUTIQUES FRANCAISES(FRANCE), 1987, vol. 45, no. 2, p163-174

## Description

La présente invention a trait à un procédé de production de substances d'origine végétale par culture "in vitro".

De nombreuses substances d'origine végétale présentent un certain intérêt économique pour des industries telles que la pharmacie (principes actifs des médicaments), la cosmétique (parfum), l'alimentaire (arômes et additifs). La production de ces substances se fait généralement par extraction à partir de plantes ramassées ou cultivées. Toutefois cette méthode présente de nombreux inconvénients, en particulier en ce qui concerne le réapprovisionnement en matière première végétale (périodicité, stabilité, qualité...).

Afin de pallier ces inconvénients, divers procédés de remplacement ont été étudiés. Parmi ceux-ci, notons le procédé de culture "in vitro" de tissus ou de cellules indifférenciées des espèces productrices. A ce sujet, citons le brevet japonais JP 57-39778 concernant la culture de cals en milieu liquide agité contenant de la cellulose, afin d'en extraire les principes actifs à l'aide d'huile de paraffine et/ou de matières grasses, ainsi que la publication de Bisson et al. (Journal of medicinal Plant Research, 1983-47-164), concernant l'extraction d'huiles essentielles d'une culture de cellules indifférenciées de camomille, par ajout au milieu nutritif de gouttelettes d'une phase grasse, dans laquelle les huiles vont s'accumuler.Cependant, utilisant des cellules indifférenciées, ces techniques restent limitées à la production de produits bien définis.

La présente invention a pour but de cumuler les avantages du système différencié à ceux de la culture "in vitro".

A cet effet, le procédé selon la présente invention est caractérisé en ce que:
- on implante un organe végétal sur un milieu de culture, et
- on le maintient sous anoxie ou hypoxie par immersion sous une couche d'huile.

De préférence, on extrait les substances produites de l'organe végétal, du milieu de culture et/ou de l'huile. On peut, pour ce faire, utiliser les techniques d'extraction traditionnelle. L'intérêt principal du procédé consiste dans la mise en culture "in vitro' d'organes végétaux producteurs des substances recherchées, et dans leur maintien en survie et en phase de production de ces substances, pendant de longues périodes.

L'huile utilisée pour maintenir l'hypoxie peut permettre d'éviter les pertes éventuelles de substances volatiles et en assurer l'extraction.

Un avantage de cette invention est de permettre la production des substances d'une manière constante et reproductible.
Un autre avantage est de pouvoir prolonger la phase de production de substances par des organes végétaux, sur des périodes beaucoup plus longues que la durée normale de production "in situ" par des plantes.

Un autre avantage est de pouvoir conserver à l'organe végétal ses caractéristiques qualitatives spécifiques, ce qui est rarement le cas des cultures cellulaires indifférenciées où des substances secondaires peuvent être produites.

De cette manière, les organes végétaux non seulement ne se nécrosent pas et survivent pendant plusieurs mois, mais continuent d'accumuler dans leurs tissus, le milieu de culture et l'huile d'hypoxie des substances telles que des huiles essentielles, de nature identique ou proche de celles produites par les méthodes traditionnelles d'extraction à partir de plantes.

Ce procédé a trait au maintien en survie d'organes végétaux, et non à la croissance et/ou à la prolifération des tissus, même si cela peut se produire dans certains cas, de façon réduite.

Parmi les critères utilisables pour apprécier la survie de l'organe végétal, nous retiendrons:
- comme critères d'aspect:
   . l'évolution de la pigmentation de l'implant
   . l'apparition de chlorose ou de brunissement (oxydation).
- comme critères morphologiques:
   . l'épanouissement floral
   . l'apparition de cals et/ou de racines

L'appréciation de ces critères est faite de différentes manières:
- de manière visuelle
- à l'aide de tests biochimiques, (test de coloration...), qui permettent de juger de la viabilité de l'organe.

Les organes végétaux mis en culture peuvent être de tout type et cultivés par partie ou en entier. Il peut s'agir, par exemple, de fragments de racines, de feuilles ou de tiges, ou encore de boutons floraux ou de fleurs épanouies.

Ces plantes ou organes peuvent être de toute origine:
- soit obtenus par récoltes sur des plantes cultivées ou ramassées. Dans ce cas, le procédé va permettre d'améliorer l'utilisation des productions végétales traditionnelles, grâce à une prolongation de la phase de biosynthèse et de production des substances recherchées.
- soit formés à partir de cultures cellulaires. Dans ce cas, le procédé va permettre une totale indépendance vis à vis de la culture traditionnelle.

Les plantes ou organes végétaux peuvent être préalablement décontaminés avant leur implantation sur le milieu de culture, afin d'éliminer les microorganismes gênants. La décontamination peut se faire par immersion des plantes ou organes, pendant quelques minutes, dans une solution de décontaminant, telle que l'eau de javel, le bichlorure de mercure, l'hypochlorite de calcium ou de sodium, ou tout autre décontaminant usuel, ou par tout autre moyen de nature chimique ou physique (Rayons X,γ ...).

Le milieu de culture utilisé doit apporter à l'organe végétal les nutriments nécessaires à sa survie en phase de production.
Ce milieu nutritif peut être sous forme semi-solide (gélose aqueuse) ou sous forme d'un liquide, agité ou non agité, afin de simplifier la mise en culture des organes végétaux. Dans ce dernier cas, la mise en culture peut se résumer à une immersion des organes décontaminés dans le milieu nutritif, suivie d'un recouvrement avec un film d'huile, afin d'éviter les échanges de surface.

Il est possible de renouveler le milieu de culture, afin de toujours apporter à l'organe les éléments nécessaires à sa survie et pour en retirer les éventuels composés diffusés (antagonistes et/ou recherchés). Le renouvellement peut être effectué de manière continue ou semi-continue.

De la même manière, il est possible de modifier ce milieu par ajout de régulateurs de croissance et/ou par ajout d'éléments susceptibles de favoriser la production des métabolites recherchés (ajout de précurseurs).

Le milieu de culture est de préférence un milieu usuel dans le domaine de la culture de tissus "in vitro", tel que les milieux de Gamborg, de Heller, ou encore de Murashige et Skoog, dont les compositions sont données dans le tableau I.
Le milieu de culture préféré selon l'invention est le milieu de Gamborg.

L'huile qui recouvre l'organe végétal peut assurer deux fonctions:
- elle joue tout d'abord le rôle d'agent de conservation, en assurant le maintien de l'hypoxie, et
- elle peut agir en tant qu'agent piégeur/extracteur des substances lipophiles recherchées.

Le choix de l'huile est lié à certains critères tels que:
- son aptitude à provoquer l'hypoxie, c'est-à-dire à ne pas, ou peu, transférer l'oxygène extérieur vers l'organe végétal et son milieu de culture,
- son pouvoir de solvant vis à vis des substances recherchées,
- son absence de toxicité vis à vis des organes végétaux.

Comme il est préférable de renouveler périodiquement l'huile, celle-ci pouvant se saturer par l'extraction des substances recherchées, un milieu d'hypoxie liquide est plus aisé à manipuler qu'un milieu solide, cependant utilisable.
L'huile peut être renouvelée de façon continue ou semi-continue.
Parmi les huiles utilisables, citons les huiles d'origine organique ou végétale, les huiles de synthèse, ainsi que toute autre huile susceptible de jouer le rôle d'agent conservateur.
L'huile préférée selon l'invention est une huile de paraffine.
L'huile peut préalablement avoir été dégazée et stérilisée. Le dégazage peut s'effectuer par mise sous vide de l'huile, les gaz qui y sont contenus s'échappant et pouvant être éventuellement remplacés par de l'azote.

Afin d'éviter une dégradation de chlorophylle qui pourrait nuire à la survie de l'organe végétal, un éclairement faible, de l'ordre de 0-400 lux peut être utilisé lors de la mise en oeuvre du procédé selon l'invention. La température de culture peut être comprise entre 18°C et 35°C. Elle est de préférence d'environ 25°C.

Le présent procédé de production de substances d'origine végétale peut être réalisé en discontinu, en semi-continu ou en continu.
L'invention est illustrée plus en détail dans les exemples suivants.
Les exemples A, B et C illustrent plus particulièrement le maintien en survie et ses critères d'appréciation. Les exemples sont précédés du tableau ci-après qui donne la composition des milieux de culture utilisés.

**Tableau I**

| composition des milieux utilisés (pH=5,8) | | | |
|---|---|---|---|
| | Milieu de Murashige et Skoog mg/l | Milieu de Gamborg mg/l | Milieu de Heller mg/l |
| Saccharose | 20000 | 20000 | 20000 |
| Agar (milieux gélosés) | 8000 | 8000 | 8000 |

| Macroéléments | | | |
|---|---|---|---|
| NH₄ NO₃ | 1650 | - | - |
| (NH₄)₂SO₄ | - | 134 | - |
| CaCl₂, H₂O | 440 | 150 | 75 |
| MgSO₄, 7H₂O | 370 | 250 | 250 |
| KCl | - | - | 750 |
| KNO₃ | 1900 | 2500 | - |
| KH₂PO₄ | 170 | - | - |
| NaNO₃ | - | - | 600 |
| NaH₂PO₄, H₂O | - | - | 125 |

| Microéléments | | | |
|---|---|---|---|
| AlCl₃, 6H₂O | - | - | 0,05 |
| CoCl₂, 6H₂O | 0,025 | 0,025 | - |
| CuSO₄, 5H₂O | 0,025 | 0,025 | - |
| FeCl₃, 6H₂O | - | - | 1,0 |
| FeSO₄, 7H₂O | 27,8 | 27,8 | 27,8 |
| Na₂ -EDTA | 37,3 | 37,3 | 37,3 |
| MnSO₄, 4H₂O | 22,3 | 10,0 | 0,1 |
| NiCl₂, 6H₂O | - | - | 0,03 |
| KI | 0,83 | 0,75 | 0,01 |
| Na₂MoO₄, 2H₂O | 0,25 | 0,25 | - |
| ZnSO₄, 7H₂O | 8,6 | 2,0 | 1,0 |
| H₃BO₃ | 6,2 | 3,0 | 1,0 |

| Vitamines | | | |
|---|---|---|---|
| Panthoténate de calcium | - | - | 1 |
| Méso-inositol | 100 | 100 | 100 |
| Acide nicotinique | 10,5 | 1 | 1 |
| Pyridonxine (vit.B₆) | 0,5 | 1 | 1 |
| Thiamine (vit. B₁) | 0,1 | 10 | 1 |
| Biotine | - | - | 0,1 |

### Exemple A

Des fragments de tiges de fenouil sont décontaminés par immersion pendant quelques minutes dans une solution d'hypochlorite de calcium, puis rincés abondamment à l'eau distillée stérile. Ces fragments sont implantés sur un milieu de Gamborg solidifié par addition d'agar agar.
Ils sont ensuite placés sous hypoxie par addition d'huile de paraffine stérilisée, puis laissés à 25°C, sous éclairement faible (200 lux).
On observe l'évolution de la pigmentation des tiges de fenouil placées sous hypoxie, en comparaison avec des tiges semblables, placées sur le même milieu nutritif, mais sans ajout d'huile, pendant une durée de 18 semaines.

Au bout de 6 semaines, toutes les tiges sous hypoxie sont encore vertes, sans traces de chlorose, alors que les tiges témoins montrent toutes un début de chlorose. L'oxydation ou chlorose totale a atteint 30% des tiges témoins au bout de 8 semaines, alors que les tiges sous hypoxie ne sont que 10% à présenter un début de chlorose, et 20% au bout de 12 semaines.
A 16 semaines, 90% des tiges témoins sont oxydées totalement contre 40% pour les tiges sous hypoxie, le reste étant encore vert.
Après 18 semaines, 100% des tiges témoins sont oxydées complètement alors que seulement 50% des tiges sous hypoxie le sont, les 50% restant présentant seulement un début de chlorose.
Notons enfin qu'au bout de 6 semaines, il n'y a plus de tiges témoins totalement vertes, alors que les tiges sous hypoxie le sont encore à 50% au bout de 16 semaines.

### Exemple B

Des boutons floraux de tubéreuse sont cueillis à trois stades différents de développement.
- Stade I:: corolle de 1-1,5 cm de longueur - pétales verts
- Stade II:: corolle de 2 cm - début de blanchissement des pétales
- Stade III:: corolle de 3 cm - pétales blancs

Ils sont décontaminés et implantés sur un milieu de Gamborg gélosé.
Ces boutons sont ensuite placés sous hypoxie par ajout d'huile de paraffine. On n'ajoute pas d'huile sur les boutons témoins. On laisse le tout sous éclairement faible (200 lux) et à 25°C.

L'évolution de l'oxydation de ces boutons floraux dans le temps est notée arbitrairement selon une échelle de 0 à 7, explicitée ci-après:
- degré 0:: absence de taches d'oxydation
- degré 1:: début d'oxydation aux extrémités des pétales, sur 10% des pétales maximum
- degré 2:: début d'oxydation aux extrémités des pétales, sur plus de 10 % des pétales
- degré 3:: oxydation de la moitié de la surface des pétales sur 10% des pétales maximum,
- degré 4:: oxydation de la moitié de la surface des pétales sur plus de 10% des pétales
- degré 5:: oxydation de la moitié de la surface des pétales sur plus de 50% des pétales
- degré 6:: oxydation importante de la fleur (80%)
- degré 7:: oxydation complète de la fleur.

Il est à noter que l'ajout d'antioxydants à l'huile de paraffine n'apporte pas de diminution notable de l'oxydation.

L'emploi d'huile dégazée ne permet pas non plus de noter une différence significative par rapport à l'huile non dégazée. Par contre, la différence entre les boutons placés sous hypoxie et les boutons témoins est nette.

Les tableaux suivants montrent l'évolution du degré d'oxydation dans le temps:

Pour les boutons de stade I:

| Temps (jours) | 2 | 5 | 7 | 10 | 13 | 17 | 18 | 24 | 28 |
|---|---|---|---|---|---|---|---|---|---|
| Témoin | 0 | 0 | 1 | 1 | 5 | 5 | 6 | 7 | 7 |
| Huile | 0 | 0 | 0 | 1 | 1 | 1 | 2 | 2 | 3 |
| Huile dégazée | 0 | 0 | 0 | 1 | 1 | 1 | 2 | 2 | 3 |

On note que les témoins sont complètement oxydés au bout de 24 jours, alors que la survie des boutons sous hypoxie se prolonge au-delà de 6 semaines.

Pour les boutons de stade II:

| Temps (jours) | 2 | 5 | 7 | 10 | 13 | 17 | 18 | 24 | 28 |
|---|---|---|---|---|---|---|---|---|---|
| Témoin | 0 | 0 | 2 | 2 | 5 | 7 | 7 | 7 | 7 |
| Huile | 0 | 0 | 0 | 1 | 1 | 1 | 1 | 2 | 2 |
| Huile dégazée | 0 | 0 | 0 | 1 | 1 | 1 | 1 | 2 | 2 |

Les témoins sont oxydés après 17 jours, les boutons sous hypoxie n'étant que faiblement oxydés après 4 semaines.

Pour les boutons de stade III:

| Temps (jours) | 2 | 5 | 7 | 10 | 13 | 17 | 18 | 24 | 28 |
|---|---|---|---|---|---|---|---|---|---|
| Témoin | 2 | 5 | 7 | 7 | 7 | 7 | 7 | 7 | 7 |
| Huile | 0 | 0 | 1 | 1 | 2 | 3 | 3 | 4 | 4 |
| Huile dégazée | 0 | 1 | 1 | 2 | 3 | 4 | 4 | 5 | 5 |

Les témoins sont oxydés au bout de 7 jours, alors que les boutons placés sous huile peuvent être conservés jusqu'à 28 jours, avant d'être totalement oxydés.

### Exemple C

Différents organes végétaux ont été décontaminés, implantés sur un milieu de Gamborg gélosé, puis placés sous hypoxie sous une couche d'huile de paraffine, et laissés à 25°C, sous éclairement faible (200 lux). Parmi ces végétaux, des plantes aromatiques (I), des plantes à parfum (II) et des plantes non productrices d'arôme, mais productrices de substances non volatiles d'intérêt pharmacologique ou cosmétique (III).

Les résultats de ces essais sont donnés dans le tableau ci-dessous:

| | Espèces | Organes | Survie possible en hypoxie |
|---|---|---|---|
| I | . Ocimum basilicum | feuilles | 10-12 semaines |
| | . Verbena triphylia | feuilles | 3-4 semaines |
| II | . Pelargonium capitatum | feuilles | 8-10 semaines |
| | . Pogostemon patchouli | feuilles | 8-10 semaines |
| III | . Ferula galbanum | racines | 10-12 semaines apparition de cals |
| | . Gingko biloba | feuilles | 14-16 semaines |

Quel que soit le type de plante ou d'organe implanté, la survie en hypoxie est possible pendant au moins un mois, et peut être maintenue sur une période pouvant atteindre 4 mois.

### Exemple 1

Des feuilles de menthe, décontaminées puis implantées sur un milieu de Gamborg, sont placées sous hypoxie sous une couche d'huile de paraffine, à 25°C.
On suit la production de composés aromatiques au cours de la survie, pendant une période de 5 semaines.
Les feuilles de menthe sont séparées en 3 lots et soumises à des conditions expérimentales différentes:
- Lot A:: éclairement fort (2000 lux)
- Lot B:: éclairement faible (200 lux)
- Lot C:: éclairement fort (2000 lux) et ajout de précurseur des terpènes.

On suit plus spécialement la présence dans l'huile d'un composé caractéristique de l'essence: la carvone.
L'huile est renouvelée régulièrement pendant une période de 35 jours, dans les 3 lots, et l'on effectue une analyse quantitative par densitométrie sur chromatographie couche mince, afin de déterminer la quantité de carvone contenue dans l'huile récoltée. Les résultats sont confirmés par une analyse par chromatographie en phase gazeuse.
Le tableau ci-dessous donne la quantité de carvone accumulée dans l'huile, pour les 3 lots, et sur une période de 5 semaines.
La quantité de carvone est donnée en microgramme par gramme de matière fraîche de feuille implantée.

| Temps (jours) | 1 | 2 | 3 | 4 | 7 | 14 | 21 | 28 | 35 |
|---|---|---|---|---|---|---|---|---|---|
| Lot A | 20 | 60 | 10 | 240 | 10 | 170 | 120 | 240 | 110 |
| Lot B | 110 | 60 | 10 | 20 | 20 | 20 | 20 | 140 | 110 |
| Lot C | 620 | 60 | 80 | 10 | 170 | 170 | 260 | 340 | 170 |

On remarque un pic de production de carvone en début de cinétique, suivi d'une baisse de production, puis d'une reprise, avec un nouveau pic d'accumulation à 28 jours.

Le tableau ci-dessous montre le gain de carvone accumulée pendant la période de 35 jours. (en µg/g de matière fraîche).

| | I | II | III | IV |
|---|---|---|---|---|
| Lot A | 690 | 980 | 635 | 925 |
| Lot B | 690 | 510 | 600 | 420 |
| Lot C | 690 | 1880 | 820 | 2010 |
| I: contenu initial en carvone de la feuille implantée II: carvone accumulée dans l'huile pendant 35 jours III: teneur finale en carvone des tissus résiduels IV: gain en carvone sur 35 jours (II + III - I) | | | | |

Ces données montrent bien la reprise de la synthèse de carvone au cours du temps, plutôt qu'un simple phénomène d'extraction solide/liquide, le total produit pouvant atteindre trois fois la quantité contenue dans l'implant initial.

### Exemple 2

Des tiges de fenouil selon l'exemple A sont cultivées in vitro sur un milieu de Gamborg gélosé et sous huile de paraffine.
On suit la production de p-allylanisole, composé caractéristique de l'essence, au cours de la survie, pendant une période de 2 mois.

Les tiges de fenouil sont séparées en 3 lots qui sont soumis à des conditions expérimentales différentes:
- Lot A:: milieu de Gamborg de base
saccharose: 20 g l⁻¹
éclairement: 200 lux
- Lot B:: milieu de Gamborg de base + régulateur de croissance
saccharose: 20 g l⁻¹
éclairement: 200 lux
- Lot C:: 1/10 milieu de Gamborg de base
saccharose: 10 g l⁻¹
éclairement: 200 lux

L'huile de paraffine est régulièrement renouvelée à 1, 3, 5 et 7 semaines, puis analysée quantitativement et qualitativement par chromatographie en phase gazeuse. On note la présence de p-allylanisole dans l'huile ainsi que l'apparition de nombreux autres composés de nature aromatique.

Le tableau suivant donne la quantité de p-allylanisole accumulée dans l'huile (en µg/g de matière fraîche de feuille implantée), en fonction du temps:

| | Temps (jours) | | | | | | | |
|---|---|---|---|---|---|---|---|---|
| | | | | | I | II | III | IV |
| | 7 | 21 | 35 | 49 | | | | |
| Lot A | 222 | 42 | 122 | traces | 333 | 386 | 35 | 88 |
| Lot B | 400 | 48 | 42 | traces | 333 | 490 | 30 | 187 |
| Lot C | 291 | 94 | 52 | 136 | 333 | 573 | 106 | 346 |
| I: contenu initial en p-allyanisole de la tige implantée II: p-allylanisole accumulé dans l'huile III: teneur finale en p-allylanisole des tissus résiduels IV: gain en p-allylanisole sur 7 semaines (II + III-I) | | | | | | | | |

La production totale peut atteindre le double de celle de l'implant (Lot C).

## Revendications

1. Procédé de production de substances d'origine végétale par culture "in vitro", caractérisé en ce que:
- on implante un organe végétal sur un milieu de culture, et
- on le maintient sous hypoxie par immersion sous une couche d'huile.

2. Procédé selon la revendication 1, caractérisé en ce que l'on extrait les substances d'origine végétale produites de l'organe végétal, du milieu de culture et/ou de l'huile.

3. Procédé selon la revendication 1, caractérisé en ce que le milieu de culture et/ou l'huile sont renouvelés de façon continue ou semi-continue.

4. Procédé selon la revendication 1, caractérisé en ce que l'huile est une huile d'origine organique, une huile d'origine végétale ou une huile de synthèse, et qu'elle est stérilisée et/ou dégazée.

5. Procédé selon la revendication 4, caractérisé en ce que l'huile est une huile de paraffine.

6. Procédé selon la revendication 1, caractérisé en ce que l'organe végétal est tout ou partie d'une racine, d'une feuille, d'une fleur, ou d'un bouton floral.

7. Procédé selon la revendication 1, caractérisé en ce que l'organe végétal est préalablement décontaminé avant implantation.

8. Procédé selon la revendication 1, caractérisé en ce que l'on ajoute des précurseurs et/ou des régulateurs de croissance au milieu de culture.

9. Procédé selon la revendication 1, caractérisé en ce que l'on maintient l'organe sous hypoxie sous un éclairement compris entre 0 et 400 lux.

10. Procédé selon la revendication 1, caractérisé en ce que l'on maintient l'organe sous hypoxie à une température comprise entre 18 et 35°C.

## Claims

1. A process for the production of substances of vegetable origin by "in vitro" culture, characterized in that
- a vegetable organ is implanted in a culture medium and
- is kept under hypoxia by immersion beneath a layer of oil.

2. A process as claimed in claim 1, characterized in that the substances of vegetable origin produced are extracted from the vegetable organ, the culture medium and/or the oil.

3. A process as claimed in claim 1, characterized in that the culture medium and/or the oil are renewed continuously or semi-continuously.

4. A process as claimed in claim 1, characterized in that the oil is an oil of organic origin, an oil of vegetable origin or a synthesis oil and in that it is sterilized and/or degassed.

5. A process as claimed in claim 4, characterized in that the oil is a paraffin oil.

6. A process as claimed in claim 1, characterized in that the vegetable organ is all or part of a root, leaf, flower or flower bud.

7. A process as claimed in claim 1, characterized in that the vegetable organ is decontaminated before implantation.

8. A process as claimed in claim 1, characterized in that precursors and/or growth regulators are added to the culture medium.

9. A process as claimed in claim 1, characterized in that the organ is kept under hypoxia in low light of 0 to 400 lux.

10. A process as claimed in claim 1, characterized in that the organ is kept under hypoxia at a temperature in the range from 18 to 35°C.

## Patentansprüche

1. Verfahren zur Erzeugung von Pflanzensubstanzen durch In-vitro-Kultur, dadurch gekennzeichent, daß
- ein pflanzliches Organ in ein Kulturmedium implantiert wird und
- durch Versenken unter eine Ölschicht unter Hypoxie gehalten wird.

2. Verfahren nach Anspruch 1, dadurch gekennzeichent, daß die vom pflanzlichen Organ erzeugte Pflanzensubstanz aus dem Kulturmedium und/oder dem Öl extrahiert wird.

3. Verfahren nach Anspruch 1, dadurch gekennzeichnet, daß das Kulturmedium und/oder das Öl kontinuierlich oder halbkontinuierlich erneuert werden.

4. Verfahren nach Anspruch 1, dadurch gekennzeichnet, daß das Öl ein Öl organischen Ursprungs, ein Öl pflanzlichen Ursprungs oder ein Syntheseöl ist und daß es sterilisiert und/oder entgast wird.

5. Verfahren nach Anspruch 4, dadurch gekennzeichnet, daß das Öl Paraffinöl ist.

6. Verfahren nach Anspruch 1, dadurch gekennzeichnet, daß das pflanzliche Organ eine Wurzel, ein Blatt, eine Blüte oder eine Blütenknospe oder ein Teil davon ist.

7. Verfahren nach Anspruch 1, dadurch gekennzeichnet, daß das pflanzliche Organ vor seiner Implantierung dekontaminiert wird.

8. Verfahren nach Anspruch 1, dadurch gekennzeichnet, daß dem Kulturmedium Vorläufer und/oder Wachstumsregler beigegeben werden.

9. Verfahren nach Anspruch 1, dadurch gekennzeichnet, daß das Organ unter einer Beleuchtung von 0 bis 400 Lux unter Hypoxie gehalten wird.

10. Verfahren nach Anspruch 1, dadurch gekennzeichnet, daß das Organ bei einer Temperatur von 18 bis 35°C unter Hypoxie gehalten wird.
